# EUROPEAN PATENT SPECIFICATION

(11) **EP 0 728 100 B1**
(45) Date of publication and mention of the grant of the patent: **29.12.1999**
(21) Application number: 94927231.4
(22) Date of filing: 29.08.1994
(51) Int. Cl.: B64B 1/22

(54) **A CARGO COMPARTMENT FOR A LIGHTER-THAN-AIR VEHICLE**
FRACHTRAUM EINES LUFTSCHIFFES
SOUTE DE DIRIGEABLE

(30) Priority: 08.11.1993 US 148224
(43) Date of publication of application: 28.08.1996
(73) Proprietor: Lockheed Martin Corporation, Bethesda, MD 20817 (US)
(72) Inventor: BELIE, Robert, G., Moorpark, CA 93021 (US); NICOLAI, Leland, M., Castaic, CA 91348 (US)
(74) Representative: Boon, Graham Anthony
(86) International application number: PCT/US94/09611
(87) International publication number: WO 95/13213

(56) References cited:
- WO-A-88/00906
- WO-A-93/24364
- DE-A- 3 508 100
- US-A- 3 486 719
- US-A- 5 143 323

## Description

### BACKGROUND OF THE INVENTION

### Field of the Invention

The invention relates to the field of lighter-than-air vehicles and, in particular, to such vehicles having cargo compartments.

### Description of Related Art

Lighter-than-air vehicles have either non-rigid or rigid gasbags. There are three general types of non-rigid vehicles: those comprising a single gas filled bag; several gasbags joined together in series; and, of course, those having a multiple number gasbags within a non-rigid envelope. Rigid airships have an internal structure defining the shape of the vehicle containing a plurality of gasbags with an aerodynamic cover there over. One of the problems with both rigid and non-rigid lighter-than-air vehicles is their limited ability to station keep and/or maneuver when docking, especially if there are any significant cross-winds. This is primarily due to their large cross-sectional area, which causes the vehicle to "weather vane" and "wave" with the wind. They are particularly difficult to control if the wind is gusting or when there are significant up or down drafts. In fact, docking has proven to be the most difficult portion of a flight for a lighter-than-air-vehicle. Thus the most common docking procedure is moor the vehicle by its nose and let it weather vane.

In non-rigid designs the cargo flight station and cargo compartments are suspended from the bottom of the vehicle in what is most often referred to as a gondola. In rigid designs, it is also common practice to mount the flight station/passenger and cargo compartments under the gasbag. Unlike a conventional aircraft, the lift forces developed by the gasbag remains when docked. Thus as cargo is unloaded, the net lift force increases. In the past, this increase in net lift has been absorbed by docking restraints, for example mooring lines. If the cargo weight is very large, the force exerted on the mooring lines also becomes large and "station keeping" when docked becomes even more difficult. Therefore, unloading and reloading of cargo sometimes must wait until any winds have sufficiently died down or additional mooring lines must be used to "lock" the vehicle in one position. It is therefore obvious that it is always important to minimize the time spent in such operations.

Thus conventional loading of cargo containers and the like through doors located on the side of the vehicle is too slow and it would be difficult to simultaneously load and unload cargo in order to maintain the gross weight of the vehicle constant. Cargo aircraft, such as the C-5 military transport, have front and rear opening doors to the cargo compartment. However, moving the cargo simultaneously in the front and out the rear of a large lighter-than-air vehicle would still be a difficult and time consuming operation, especially if the cargo compartment is some 183 to 234 m (600 to 800 feet) long. In addition, requiring an uninterrupted passageway over such a length would greatly complicate the design of the gondola. The use of individual cargo compartments located along either side of the vehicle can reduce the cargo loading and unloading time, but does not address the need to maintain a constant payload weight on the vehicle. In U.S. Patent No. 5,143,323, "Airship Handling System" by F. Husain, et al. a vehicle is disclosed having a cargo/passenger module that is secured in a recess in the gondola. However, properly securing such a module to the gondola is believed to complicate the design of the gondola. In addition, the cargo module would have to be flight certified increasing its cost. In fact, no prior art cargo compartment design has addressed the need to maintain a constant payload weight during loading and unloading of cargo and/or to provide a cargo loading system that minimizes the docking time for unloading and loading cargo.

Attention is also directed to PCT/WO93/24364 which is prior art under Article 54(3) only when considering novelty and which describes a lighter-than-air vehicle provided wit a cargo-carrying structure mounted to the bottom of the vehicle.

It is a primary object of the invention to provide a lighter-than-air vehicle having a cargo compartment that allows a constant neutral buoyancy to be maintained on the vehicle during loading and unloading thereof.

It is another primary object of the invention to provide a lighter-than-air vehicle having a cargo compartment that provides for simultaneous loading and unloading of cargo while maintaining a constant neutral buoyancy on the vehicle.

It is a further object of a preferred embodiment of the invention to provide a lighter-than-air vehicle having a cargo compartment wherein the cargo can be loaded and unloaded using individual cargo containers.

### SUMMARY OF THE INVENTION

The invention is a lighter-than-air vehicle having a cargo compartment, the vehicle having a longitudinal, a vertical and a lateral axis. The cargo compartment includes a cargo carrying structure mounted to the bottom of the vehicle and which extends along at least a portion of the longitudinal axis thereof. The cargo compartment has a plurality of passageways. Each of the passageways is aligned with the lateral axis of the vehicle and extends completely through the cargo compartment. The passageways are adapted to simultaneously off load cargo from one end and to on load cargo from the other. Preferably each passageway is adapted to receive at least one cargo container.

A cargo container moving system is preferably mounted in the floor of each passageway for moving the at least one cargo container in one end and out the opposite end of the passageway and to intermediate positions therebetween. The container moving system includes a plurality of powered rollers mounted in the floor of each of the passageways. In addition, the cargo container moving system further includes a plurality of non-powered (passive) rollers mounted in the floor of each of the passageways for movably supporting the at least one cargo container therein.

A cargo container securing system is preferably provided for releasable securing the at least one cargo container within the passageway. The cargo container is provided with a flange along the bottom of the front and rear ends thereof. The cargo securing system further includes a pair of guide rails mounted in a spaced relationship on the floor of the passageway. Thus as the cargo containers are moved into and out of the passageway and to intermediate positions therebetween, the rails engage the flanges on the cargo containers and restrain them from movement along the longitudinal axis of the vehicle. The cargo container securing system further includes a locking system to releasable secure the cargo containers in the intermediate positions along the floor of the passageway preventing movement along the vertical and lateral axis. Although the above described cargo moving and securing systems are preferred, other systems are useable. For example cargo pallets restrained by cargo nets tied down to the passageway floor in combination or air cushion systems for movably supporting the pallets instead of a passive roller system.

### BRIEF DESCRIPTION OF THE DRAWINGS

Figure 1 is a perspective view of a lighter-than-air vehicle, incorporating the subject cargo compartment, shown in the docked position.
Figure 2 is partial perspective view partially broken away of the vehicle shown in Figure 1 providing an interior view of the subject cargo compartment.
Figure 3 is partial cross-sectional view of the vehicle shown in Figure 1 taken along the line 3-3, providing a side view of the subject cargo compartment.
Figure 4 is a view similar to Figure 3 illustrating the loading and unloading of cargo containers.
Figure 5 is an enlarged perspective view of a cargo container tie down device used to secure cargo containers with the cargo compartment.

### DESCRIPTION OF THE PREFERRED EMBODIMENT

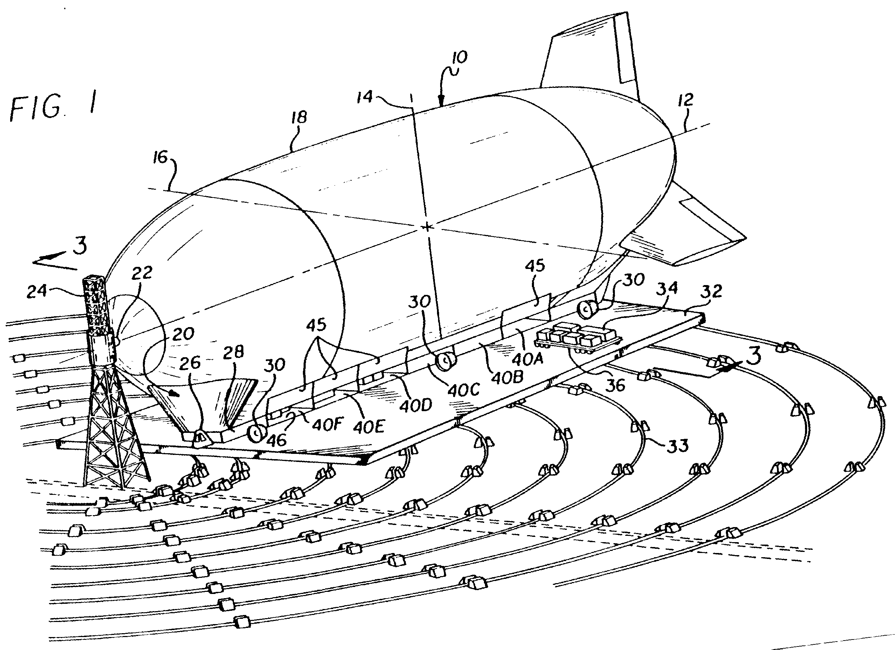

Illustrated in Figure 1 is a lighter-than-air vehicle incorporating the subject cargo compartment. The vehicle, designated by numeral 10, is a non-rigid design having a longitudinal axis 12, vertical axis 14, and lateral axis 16 and includes a helium bag 18 with a gondola 20, mounted at the bottom. The vehicle 10 is shown in a docked position tethered by its nose 22 to a tower 24. Thus it is free to weather vane in the wind. The gondola 20 includes a flight station 26, and cargo compartment 28 upon which are mounted propulsion systems 30. Whereas a gondola type cargo compartment (suspended cargo compartment) is necessary on a non-rigid vehicle, it is certainly not required on a rigid design. Thus a gondola style cargo compartment is also for purposes of illustration only. A movable platform 32 mounted on a plurality of circularly configured rails 33 is positioned under the vehicle 10 and is movable in a circle about the tower 24. A plurality of cargo containers 34, mounted on a portable loading cart 36, are positioned to be loaded into the cargo compartment 28. Thus the platform 32, with cargo containers 34 thereon, can rotate under power with the vehicle 10, if it "weather vanes".

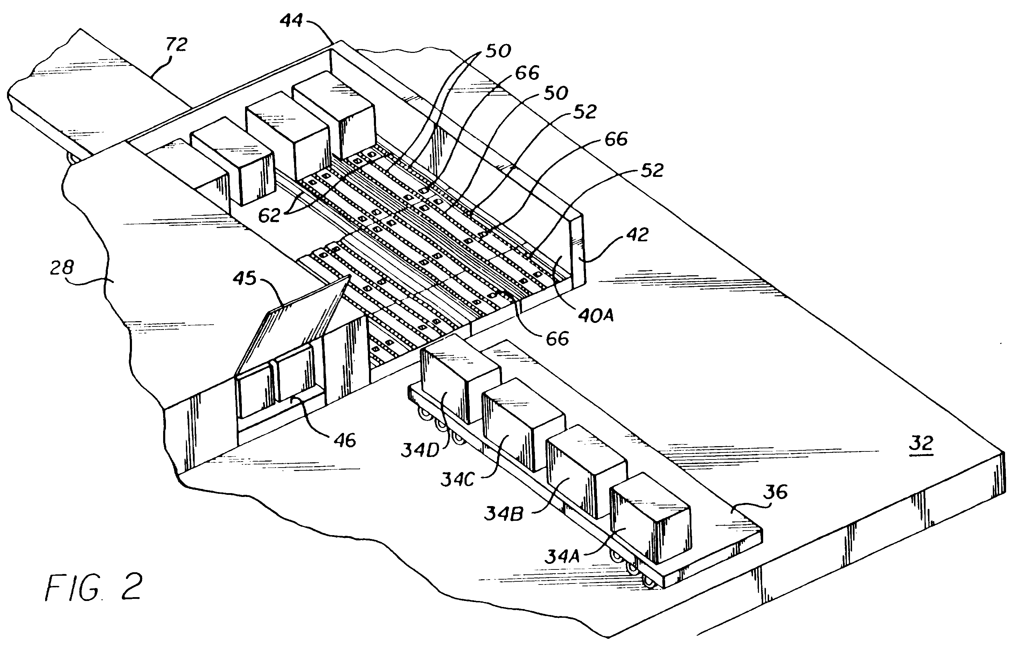

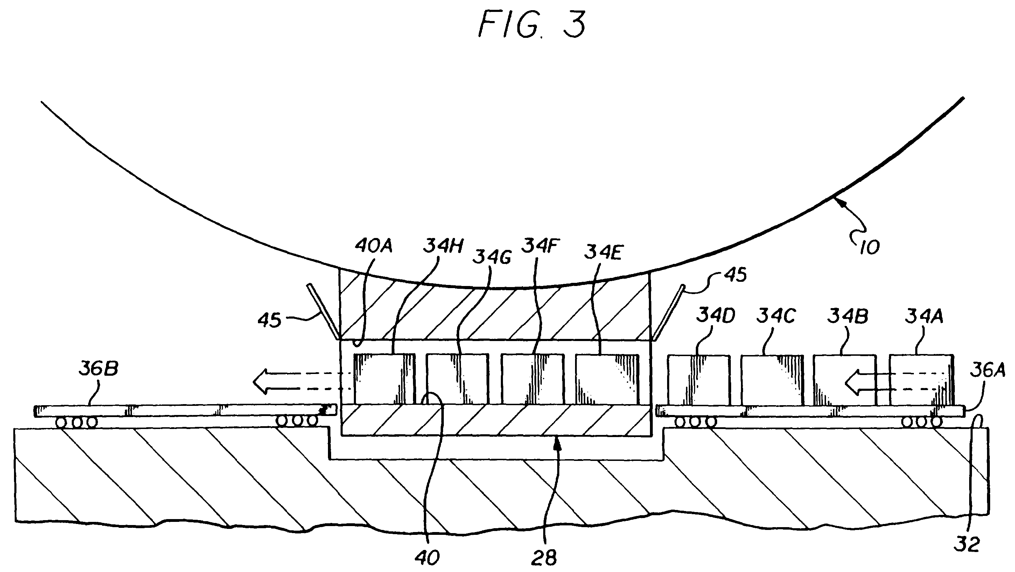

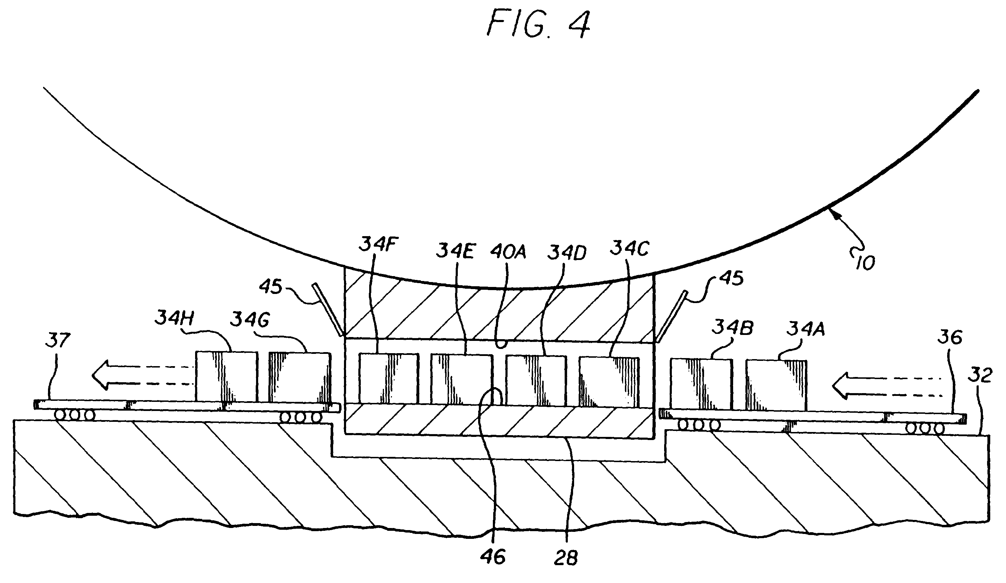

Still referring to Figure 1 and additionally to Figures 2 through 5, it can be seen that the cargo compartment 28 includes a plurality of passageways 40A-F extending along the longitudinal axis 12. The passageway 40A-F are aligned with the lateral axis 16 and extend completely through the cargo compartment with first and second ends 42 and 44, respectively, having fold up doors 45 attached thereto. The passageways are configured to receive and hold one or more of the cargo containers 34. Because all the passageways 40A-F are basically identical the following remarks shall be directed at just one, 40A. The floor 46 of the passageway 40A contains a plurality of roller assemblies 50 that allow the cargo containers 34 to be "rolled" on to the floor 46 and, in fact, in end 42 when loading and out end 44 when unloading. Although the cargo containers 34 can be pushed or pulled by hand, a plurality of powered roller assemblies 52 are provided in the floor 46 to assist in the movement of the cargo containers 34 in and out of the passageway 40A and intermediate positions therebetween. A suitable powered roller assembly is manufactured by Lucus Western, Incorporated, Electric Systems Division, Brea, California.

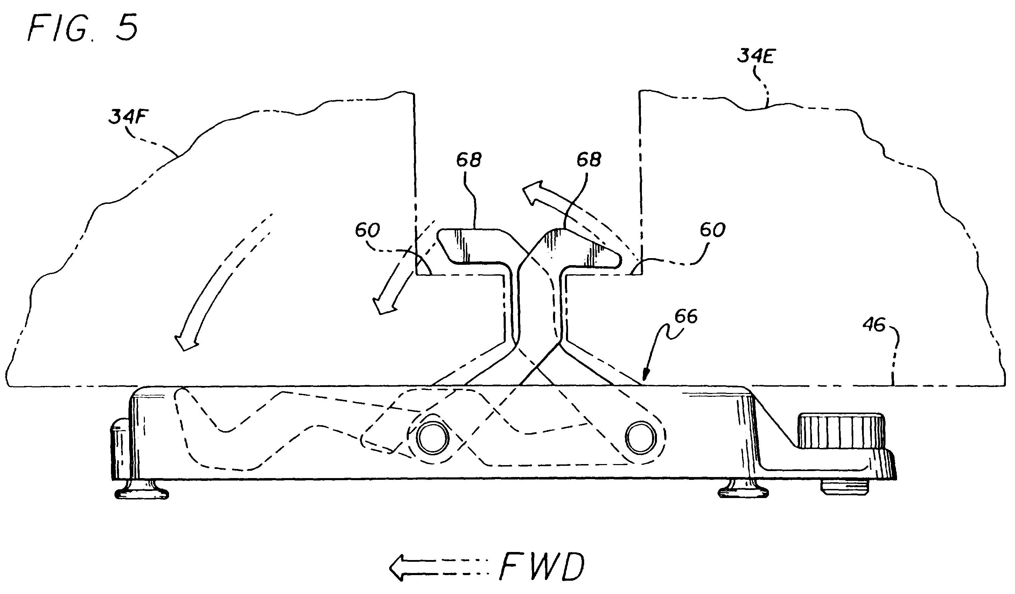

Referring to Figure 5, to lock the cargo container 34 within the passageway 46A, the container is provided with flanges 60 on the ends thereof. These flanges 60 are adapted to slidably engage guide rails 62 mounted on the floor 46. Thus once the flanges 60 engage the rails 62, the cargo container is restrained from moving along the longitudinal axis 14 of the vehicle (forwards and backwards). Additionally, retractable cargo restraints 66 are provided to restrain the containers 34 movement along the vertical and lateral axis, 14 and 16, respectively (upwards, downwards and from side to side). A suitable cargo restraints 66 can be obtained from Ancra, Corporation, El Segundo, California. a combination passive roller tray and cargo restraint is manufactured by Pemco Engineering, Stanton, California. Referring particularly to Figure 5 it can be seen that the cargo restraint 66 includes two restraining "hooks" 68 that engage the flanges 60 on two adjacent cargo containers 34. Note again that this particular restraint is only illustrative and there are numerous other types that may be used in this application.

Referring back to figures 1-4, in operation, the vehicle 10 is first docked with the nose 22 moored to the tower 24 and positioned over the platform 32. The vehicle 10 is secured to the platform 32 by mooring lines (not shown). Cargo containers 34A-D are positioned on a cart 36 in proximity to the end 42 of the passageway. The cart 36 includes cargo moving systems similar to those found in the passageways 40. An empty cart 72 is shown positioned on the opposite end 44 of the passageway 40. With the doors 45 opened, the cargo containers 34A-D are moved off the cart 36 into the passageway 40, while cargo containers 34E-H are simultaneously removed from the passageway onto cart 72. Note that, while only one cart 36 is shown, it is envisioned that all the passageways 40A-F would be simultaneously unloaded and loaded. Thus a constant payload weight is always maintained, balancing the lift forces generated by the airbag 18. Of course, the individual weight of the loaded cargo containers must be known so that they can be matched with the container being unloaded. In addition, the normal center of gravity shifts must be considered, as in the case of conventional aircraft. If the total weight of the new cargo is dramatically different from that being off loaded, then containers loaded with ballast (not shown) may have to be used. Such a case would occur if the cargo being unloaded were automobiles and cut flowers were being loaded.

While the invention has been described with reference to a particular embodiment, it should be understood that the embodiment is merely illustrative as there are numerous variations and modifications which may be made by those skilled in the art. Thus, the invention is to be construed as being limited only by the scope of the appended claims.

## Claims

1. A lighter-tan-air vehicle (10) having a longitudinal axis (12), a vertical axis (14) and a lateral axis (16), the vehicle being provided with a cargo compartment (28) comprising:
a cargo carrying structure mounted to the bottom of the vehicle, said structure extending along at least a portion of the longitudinal axis (12) of the vehicle, characterised by said structure having a plurality of open ended passageways (40A-F), each having a floor (46), each of said passageways being aligned with the lateral axis (16) of the vehicle and extending completely through said structure, said passageways being adapted to simultaneously off load cargo from one end of said passageway and to on load cargo from the opposite end.

2. A vehicle as set forth in claim 1, comprising first means (50, 52) mounted in each passageway (40A-F) for moving the cargo in one end and out the opposite end of said passageway and intermediate positions therebetween.

3. A vehicle as set forth in claim 2, comprising second means (62, 66, 68) for releasably securing the cargo within said passageway.

4. A vehicle as set forth in claim 3, wherein the cargo is mounted within at least one container (34), and wherein said first means (50, 52) is adapted to move the at least one cargo container (34) in one end and out the opposite end of said passageway and intermediate positions therebetween; and said second means (62, 66, 68) is adapted to releasably secure the at least one cargo container (34) within said passageway.

5. A vehicle as set forth in claim 4, wherein said first means (50, 52) is mounted on the floor of each passageway.

6. A vehicle as set forth in claim 5, wherein said second means (62, 66, 68) is mounted on the floor of each passageway.

7. A vehicle as set forth in claim 6, wherein said first means (50, 52) includes a plurality of powered rollers (52) mounted in said floor of each of said passageways for moving the at least one cargo container in one end and out the other end of said passageway and intermediate positions therebetween.

8. A vehicle as set forth in claim 7, wherein said first means (50, 52) further comprises a plurality of non-powered rollers (50) mounted in said floor of each of said passageways for movably supporting the at least one cargo container (34).

9. A vehicle as set forth in claim 4, 5, 6 or 7, wherein said second means includes a pair of guide rails (62) mounted in a spaced relationship on said floor of said passageway, such that, as the at least one cargo container (34) is moved into and out of said passageway and to said intermediate positions therein, said rails engage the at least one cargo container and restrain it from movement along the longitudinal axis of the vehicle.

10. A vehicle as set forth in claim 9, wherein said second means includes locking means (66, 68) to releasably secure the at least one cargo container (34) in said intermediate positions along said floor of said passageway from movement along the vertical and lateral axis of the vehicle.

11. A vehicle as set forth in claim 10, further comprising doors (45) mounted on each end of said passageway for sealing of the interior thereof.

## Patentansprüche

1. Luftfahrzeug (10), das eine Longitudinalachse (12), eine Vertikalachse (14) und eine Lateralachse (16) aufweist, wobei das Fahrzeug mit einem Frachtabteil (28) versehen ist, mit:
einem frachttragenden Aufbau, der an der Unterseite des Fahrzeuges angebracht ist, wobei sich der Aufbau entlang zumindest eines Abschnitts der Longitudinalachse (12) des Fahrzeugs ausdehnt,
dadurch gekennzeichnet, daß
der Aufbau eine Vielzahl von Durchgängen (40A-F) mit offenen Enden aufweist, von denen jeder einen Boden (46) aufweist, jeder der Durchgänge mit der Lateralachse (16) des Fahrzeuges ausgerichtet ist und sich komplett durch den Aufbau ausdehnt,
die Durchgänge in der Lage sind, gleichzeitig Fracht von einem Ende des Durchgangs auszuladen und von dem gegenüberliegenden Ende Fracht einzuladen.

2. Fahrzeug nach Anspruch 1, das erste Mittel (50, 52) aufweist, die an jedem Durchgang (40A-F) zum Bewegen der Fracht in ein Ende und aus einem gegenüberliegenden Ende des Durchgangs und dazwischenliegenden Positionen angebracht ist.

3. Fahrzeug nach Anspruch 2, das zweite Mittel (62, 66, 68) zum wiederlösbaren Sichern der Fracht in dem Durchgang aufweist.

4. Fahrzeug nach Anspruch 3, bei welchem die Fracht in mindestens einem Container (34) angebracht ist, und bei welchem das erste Mittel (50, 52) in der Lage ist, zumindest einen Frachtcontainer (34) in ein Ende und aus dem gegenüberliegenden Ende des Durchgangs und dazwischenliegender Positionen zu bewegen; und das zweite Mittel (62, 66, 68) in der Lage ist, mindestens einen Frachtcontainer (34) mit dem Durchgang wiederlösbar zu sichern.

5. Fahrzeug nach Anspruch 4, bei welchem das erste Mittel (50, 52) auf dem Boden des Durchgangs angeordnet ist.

6. Fahrzeug nach Anspruch 5, bei welchem das zweite Mittel (62, 66, 68) auf dem Boden jedes Durchgangs angebracht ist.

7. Fahrzeug nach Anspruch 6, bei welchem das erste Mittel (50, 52) eine Vielzahl von angetriebene Rollen (52) enthält, die an dem Boden jedes der Durchgänge zum Bewegen mindestens eines Frachtcontainers in ein Ende und aus einem Ende des Durchgangs und dazwischenliegenden Positionen angebracht ist.

8. Fahrzeug nach Anspruch 7, bei welchem das erste Mittel (50, 52) weiterhin eine Vielzahl von nicht angetriebenen Rollen (50) aufweist, die in dem Boden eines jeden der Durchgänge zum beweglichen Unterstützen mindestens eines Frachtcontainers (34) angebracht sind.

9. Fahrzeug nach einem der Ansprüche 4, 5, 6 oder 7, bei welchem das zweite Mittel ein Paar von Führungsschienen (62) enthält, die in einer beabstandeten Beziehung auf dem Boden des Durchgangs derart angebracht sind, daß mindestens ein Frachtcontainer (34) in und aus dem Durchgang und den darin befindlichen Zwischenpositionen bewegt werden kann, wobei die Schienen mit zumindest einem Frachtcontainer in Eingriff stehen und von einer Bewegung entlang der Longitudinalachse des Fahrzeuges zurückgehalten werden.

10. Fahrzeug nach Anspruch 9, bei welchem das zweite Mittel ein Verriegelungsmittel (66, 68) enthält, um zumindest einen Frachtcontainer (34) in den dazwischenliegenden Positionen entlang des Bodens des Durchgangs von einer Bewegung entlang der vertikalen und lateralen Achse des Fahrzeugs wiederlösbar zu sichern.

11. Fahrzeug nach Anspruch 10, das weiterhin Türen (54) aufweist, die an einem Ende des Durchgangs zum Verschließen seines Inneren angebracht sind.

## Revendications

1. Véhicule plus léger que l'air (10) ayant un axe longitudinal (12), un axe vertical (14) et un axe latéral (16), le véhicule étant muni d'une soute (28) comportant :
une structure de support de charge montée sur la partie inférieure du véhicule, ladite structure s'étendant au moins le long d'une partie de l'axe longitudinal (12) du véhicule, caractérisée en ce que ladite structure comporte une pluralité de passages à extrémités ouvertes (40A-40F), ayant chacun un plancher (46), chacun desdits passages étant aligné avec l'axe latéral (16) du véhicule et s'étendant complètement à travers ladite structure, lesdits passages étant adaptés pour simultanément décharger une cargaison à partir d'une première extrémité dudit passage et pour charger une cargaison à partir de l'extrémité opposée.

2. Véhicule selon la revendication 1, comportant des premiers moyens (50, 52) montés dans chaque passage (40A-40F) pour faire entrer une cargaison dans une première extrémité du passage et la sortir par l'extrémité opposée dudit passage et la déplacer vers des positions intermédiaires entre celles-ci.

3. Véhicule selon la revendication 2, comportant des seconds moyens (62, 66, 68) pour fixer de manière libérable la cargaison dans ledit passage.

4. Véhicule selon la revendication 3, dans lequel la cargaison est montée dans au moins un container (34), et dans lequel lesdits premiers moyens (50, 52) sont adaptés pour faire entrer le au moins un container de cargaison (34) par une première extrémité et le sortir par l'extrémité opposée dudit passage et le déplacer vers des positions intermédiaires entre celles-ci, et lesdits seconds moyens (62, 66, 68) sont adaptés pour fixer de manière libérable le au moins un container de cargaison (34) dans ledit passage.

5. Véhicule selon la revendication 4, dans lequel lesdits premiers moyens (50, 52) sont montés sur le plancher de chaque passage.

6. Véhicule selon la revendication 5, dans lequel lesdits seconds moyens (62, 66, 68) sont montés sur le plancher de chaque passage.

7. Véhicule selon la revendication 6, dans lequel lesdits premiers moyens (50, 52) comportent une pluralité de rouleaux motorisés (52) montés dans ledit plancher de chacun desdits passages pour faire entrer le au moins un container de cargaison par une première extrémité et le sortir par l'autre extrémité dudit passage et le déplacer vers des positions intermédiaires entre celles-ci.

8. Véhicule selon la revendication 7, dans lequel lesdits premiers moyens (50, 52) comportent une pluralité de rouleaux non motorisés (50) montés dans ledit plancher de chacun desdits passages pour supporter de manière mobile le au moins un container de cargaison (34).

9. Véhicule selon la revendication 4, 5, 6 ou 7, dans lequel lesdits seconds moyens comportent deux rails de guidage (62) montés selon une disposition espacée sur ledit plancher du passage, de sorte que lorsque le au moins un container de cargaison (34) est déplacé vers l'intérieur et l'extérieur dudit passage et vers lesdites positions intermédiaires situées dans celui-ci, lesdits rails sont en contact avec le au moins un container de cargo et l'empêchent de se déplacer le long de l'axe longitudinal du véhicule.

10. Véhicule selon la revendication 9, dans lequel lesdits seconds moyens comportent des moyens de verrouillage (66, 68) destinés à fixer de manière libérable le au moins un container de cargaison (34) dans lesdites positions intermédiaires le long dudit plancher dudit passage ou vis-à-vis d'un déplacement le long des axes vertical et latéral du véhicule.

11. Véhicule selon la revendication 10, comportant de plus des portes (45) montées à chaque extrémité dudit passage pour assurer l'étanchéité de l'intérieur de celui-ci.
